Europäisches Patentamt

European Patent Office

Office européen des brevets

(11) Publication number: **0 186 783**
**B1**

## EUROPEAN PATENT SPECIFICATION

(45) Date of publication of patent specification: 26.10.88

(51) Int. Cl.⁴: **A 61 M 1/00**

(21) Application number: 85114991.4

(22) Date of filing: 26.11.85

(54) Fluid evacuator for medical use.

(30) Priority: 30.11.84 JP 251678/84

(43) Date of publication of application:
09.07.86 Bulletin 86/28

(45) Publication of the grant of the patent:
26.10.88 Bulletin 88/43

(84) Designated Contracting States:
DE FR GB IT NL SE

(56) References cited:
JP-A-59 177 055
US-A-3 018 779
US-A-3 853 128
US-A-4 073 294
US-A-4 136 696
US-A-4 266 545

(73) Proprietor: SUMITOMO BAKELITE COMPANY
LIMITED
2-2, Uchisaiwaicho 1-chome Chiyoda-ku
Tokyo 100 (JP)

(72) Inventor: Tanda, Yukitaka
Shonan Sky Haitsu 1-5-5063
3874 Oba Fujisawa-shi (JP)
Inventor: Kawai, Kenji
11-10, Kandai-2-chome
Tsujido Fujisawa-shi (JP)

(74) Representative: Henkel, Feiler, Hänzel & Partner
Möhlstrasse 37
D-8000 München 80 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

BACKGROUND OF THE INVENTION
Field of the Invention:

The present invention relates to a portable closed type fluid evacuator for medical use. More particularly, the present invention pertains to a portable closed type fluid evacuator employed to evacuate drainage fluid (body fluid) from a wound in a human body and effect adhesion of tissue in the wound.

Description of the Related Art:

It is a well known practice to use a fluid evacuator for evacuating drainage fluid (body fluid) from a wound in the body of a patient. In such a case, the distal end of a guide tube for evacuating drainage fluid is inserted into the wound, and the proximal end of the tube is connected to the fluid evacuator. The drainage fluid in the wound is evacuated by virtue of a negative pressure produced within the fluid evacuator so as to be received and accumulated therein. Known fluid evacuators, however, still suffer from various disadvantages in terms of their mechanisms or operations.

Hitherto known fluid evacuators may roughly be classified into the following three types in terms of the mechanism employed in producing a negative pressure.

The first type of fluid evacuator is known as an evacuated bottle in which the pressure within the interior of a hermetically sealed rigid container is reduced beforehand. The evacuated bottle of this type can be conveniently placed in an operative condition the instant that the associated drainage guide tube is connected to the same. Further, this evacuated bottle has a hermetic structure which involves less risk of contamination. Provision of graduation marks on the rigid container advantageously enables the quantity of fluid collected in the container to be accurately read off at any given time during the evacuation of drainage fluid. The evacuated bottle, however, has the disadvantage that, since the vacuum level is set in advance, the suction or evacuating force lowers at a linear rate in accordance with the quantity of drainage fluid being received in the container. In order to effect adhesion of tissue in wounds and thereby to quicken the healing of the wounds, it is, generally, an effective practice to maintain the interior of the wound under a constant negative pressure. A rapid drop in the negative pressure during the evacuation of drainage fluid may cause the drainage guide tube placed in the interior of the wound to be clogged with clots or other debris, and this disenables the device so that it cannot be used for further evacuation of drainage fluid. Thus, this type of fluid evacuator has the fatal drawback that the suction force may rapidly drop.

The second type of conventional fluid evacuator has a mechanism in which an expansible risilient container is previously compressed to reduce the interior volume so that a negative pressure for evacuating and accumulating drainage fluid is produced within the container utilizing the resiliency or resiliently restoring force of the container. This type of fluid evacuator has a relatively simple construction which advantageously enables a container in the form of, for example, a bellows to be mass-produced at low cost by means of blow molding. The rapid drop in suction force of this type of fluid evacuator is not so remarkable as in the case of the above-described first type but is too significant to be ignored. Further, since the container itself is deformable, when any external pressure is applied to the container during use such as, for example, when a patient equipped with the fluid evacuator ambulates, or when the device is placed on the patient's bed, the container is compressed by the external pressure, so that the vacuum level within the interior of the container may be remarkably reduced; in some cases, even a positive pressure is created in the container, which involves a risk of the evacuated and accumulated body fluid being forced back into the wound. In addition, even when the container is provided with graduation marks for the purpose of indicating the quantity of body fluid being accumulated, since the container itself is an elastic or resilient member, the fluid quantity cannot be measured with high accuracy and it is necessary to use another measuring container. Furthermore, fluid evacuators for medical treatments are generally used continuously for two or three days, maximumly about seven days, after a surgical operation. For this reason, it is always necessary to re-compress the container several times in order to newly provide it with the suction force which is required to cope with possible air leak into the wound as well as to evacuate drainage fluid. However, the fluid evacuator of this type necessitates emptying the container of the drainage fluid already accumulated therein before the container is recompressed, which imposes a disadvantageously heavy load on the nurses in charge, together with the aforementioned troublesome measurement of the quantity of accumulated drainage fluid.

The third type of conventional fluid evacuator has a mechanism in which an expansible resilient member (balloon or diaphragm) is incorporated in a rigid container and is expanded by inflation or dynamic or mechanical means so as to evacuate the rigid container, and a negative pressure for evacuating drainage fluid is produced within the container by the resiliency or deflating force of the resilient member (see, for example, the specifications of U.S. Pat. Nos. 3,809,086, 3,983,872, 4,022,209 and 3,889,677 and Japanese Patent Pre-Exam. Publication No. 177,155/1984 which was disclosed by the inventors of the present invention). This type of fluid evacuator has the following advantages. Namely, if an appropriate resilient member is employed, no rapid drop in negative pressure occurs even when drainage fluid accumulates in the container, and it is possible to maintain a substantially constant suction force at any time during evacuation of drainage fluid. In addition, since the container is rigid, it is suitable for use as a portable container. The fluid evacuator of this type, however, suffers from disadvantages similar to those of the second type. Namely, in order to re-expand the resilient member so as to provide suction force during continued use of the evacuator, it is necessary to open the container and empty the same of the accumulated drainage fluid. Further, since the resilient member is incorporated in the container, it is not possible to measure the quantity of accumulated drainage fluid during evacuation (at this time the resilient member is in an expanded position). It is therefore necessary when effecting measurement to open the container and fully deflate the resilient member. If the resilient member is expanded with the rigid container not opened to the atmosphere mistakenly, a positive pressure may be produced within the container, resulting undesirably in backflow of the drainage fluid into the wound.

SUMMARY OF THE INVENTION

In view of the above circumstances, it is a primary object of the present invention to provide a portable fluid evacuator for medical use which eliminates any rapid drop in suction force which might be caused as a result of an increase in quantity of drainage fluid being evacuated from a wound in a human body, provides a substantially constant suction force at any time during evacuation and involves no risk of drainage fluid flowing back into the wound by reason of any possible trouble occurring during use, the fluid evacuator being constituted by a rigid container which is conveniently portable, as well as enabling the quantity of accumulated drainage fluid to be directly read off with high accuracy and being hygienic and excellent in operability.

To this end, the present invention provides a fluid evacuator for medical use which comprises a first chamber for receiving and accumulating drainage fluid (body fluid) evacuated from a wound in a human body, and a second chamber for generating therein a suction force through the resiliency of an inflatable balloon member provided within a rigid container. The first and second chambers are connected at their upper portions so as to be in gas communication with each other. The first chamber is provided with a connector means for connecting thereto a guide tube for guiding body fluid, a stopper means for opening and closing the connector means, and an opening with a closure member for discharging the body fluid accumulated in the first chamber. The second chamber is provided with the inflatable balloon member and evacuating means for reducing the pressure within the second chamber. The balloon member has at least one end thereof open to the atmosphere and is mounted within the second chamber such that the interior of the balloon member is in gas communication with the atmosphere. The evacuating means includes two one-way valves and a hollow resilient member. One of the valves is operably connected to the second chamber such that gas in the interior of the second chamber flows only into the interior of the resilient member. The other valve is disposed such that gas in the interior of the resilient member flows out only to the atmosphere. The resiliency or compressive restoring force (i.e., resilient recovery force from the compressed state to the normal state of the resilient member is larger than the resiliency or deflating force of the inflatable balloon member. The resilient member is alternately compressed and released, whereby the air in the fluid evacuator is discharged to the atmosphere so that the interior of the container is provided with a negative pressure and the balloon member located within the second chamber is inflated, thereby allowing a constant negative pressure to be maintained at all times by means of the resiliency of the balloon member.

The above and other objects, features and advantages of the present invention will become clear from the following description of the preferred embodiments thereof, taken in conjunction with the accompanying drawings.

BRIEF DESCRIPTION OF THE DRAWINGS

Fig. 1 shows the external appearance of one embodiment of the fluid evacuator according to the present invention;

Fig. 2 is a sectional view of the embodiment shown in Fig. 1; and

Fig. 3 is a sectional view of another embodiment of the fluid evacuator according to the present invention.

DESCRIPTION OF THE PREFERRED EMBODIMENTS

The fluid evacuator for medical use according to the present invention will be described hereinunder in detail by way of embodiments and with reference to the accompanying drawings.

As illustrated in the drawings, the fluid evacuator according to the present invention comprises two chambers which are connected such as to be in gas communication with each other.

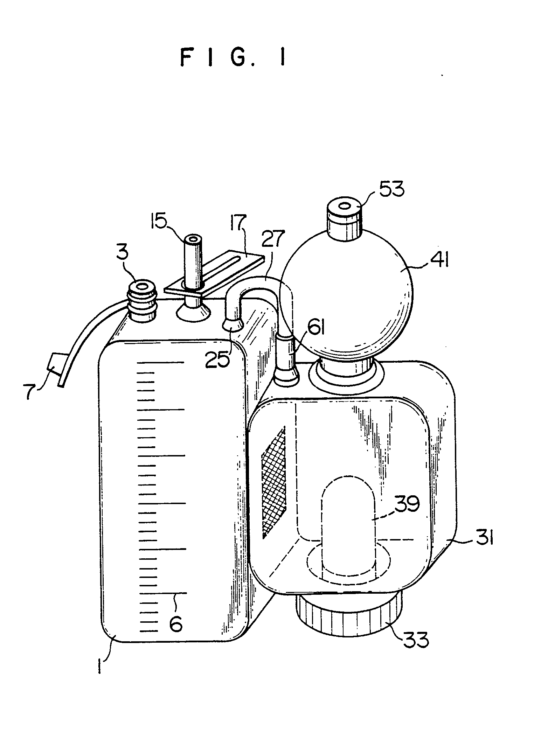

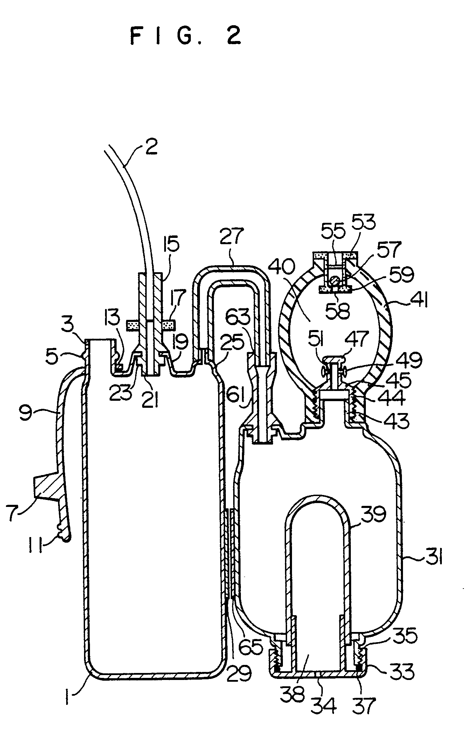

In accordance with one embodiment, which is shown in Figs. 1 and 2, the two chambers of the fluid evacuator are independently constituted by rigid containers, that is, a fluid receiving bottle 1 and an evacuation bottle 31, which are connected at their upper portions by a connecting tube 27 to be in gas communication with each other.

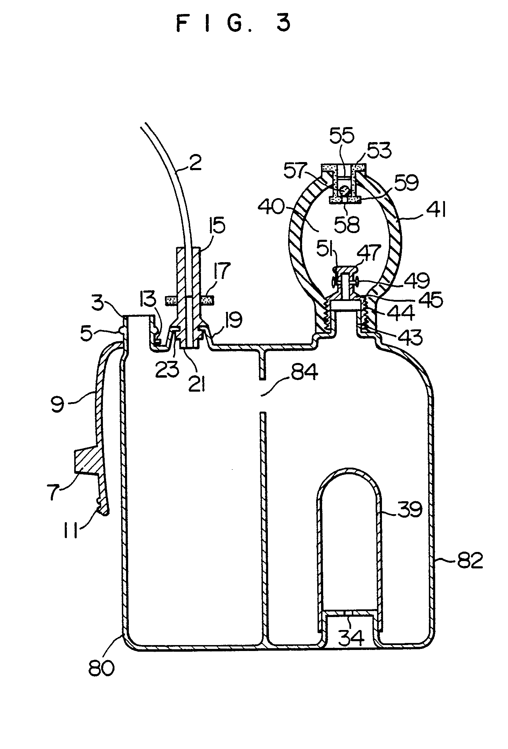

In accordance with another embodiment, which is shown in Fig. 3, the two chambers of the fluid evacuator are respectively constituted by a fluid receiving chamber 80 and an evacuation chamber 82 which are integrally formed in a single rigid container. In this case, the fluid receiving chamber 80 and the evacuation chamber 82 may be connected so as to be in gas communication with each other by providing a communicating opening 84 in the partition wall therebetween, or by employing a connecting tube in a manner similar to that in the first embodiment shown in Figs. 1 and 2.

In the first embodiment, the rigid containers may have cylindrical, spherical or parallelepiped configurations. It is, however, preferable to design them in a compact form so that the fluid evacuator may be conveniently portable. The fluid receiving bottle 1 is preferably formed such as to have a relatively narrow oblong configuration such as to facilitate measurement of the quantity of drainage fluid received in the bottle 1. The evacuation bottle 31 incorporates an inflatable balloon 39. The bottle 31 is preferably formed such as to have a spherical or parallelepiped configuration so that the balloon 39 when inflated experiences minimum contact with the inner walls of the bottle 31 whereby the balloon 39 can be smoothly inflated and may effectively perform the pressure-regulating function. It is also preferable to adopt the above-described configuration for the embodiment shown in Fig. 3 in which the two chambers are defined in an integrally molded rigid container.

The rigid containers used in accordance with the present invention may be molded from a relatively hard plastic or glass and are preferably transparent or translucent so that it is possible to check or confirm the properties and quantity of accumulated drainage fluid and the inflated condition of the balloon 39. It is preferable to employ as the constituent material of the rigid containers a material which is lightweight and tough, e.g., a hard vinyl chloride resin, so that the fluid evacuator is portable and not easily broken or destroyed during use such as, for example, when a patient equipped with it ambulates.

Various portions of the fluid evacuator according to the present invention will next be described in detail with reference to Fig. 2.

The fluid receiving bottle 1 receives and accumulates drainage fluid evacuated from a wound, while the evacuation bottle 31 is a suction pressure generator and has the function of enabling regulation of the suction pressure which has previously been set at a negative value. The fluid receiving bottle 1 is provided with a connector means for connecting a drainage fluid guide tube 1, an opening (fluid outlet port) 3 for discharging drainage fluid received and accumulated in the bottle 1, and a connecting passage which connects the bottles 1 and 31 at their upper portions so that they are in gas communication with each other. The evacuation bottle 31 incorporates the inflatable balloon 39 and is provided with a means for evacuating the interior of the fluid evacuator. The bottle 31 also has means for attaching thereto the connecting tube 27 which connects the bottles 1 and 31 so as to be in gas communication with each other. It is preferable to provide graduation marks 6 on the front side of the fluid receiving bottle 1 by means of printing, or by forming lines in the form of ridges or recesses, so that the quantity of accumulated drainage fluid can be measured. Thus, it is possible to read off the quantity of drainage fluid being evacuated at any time during use.

The connector means, which is provided on the fluid receiving bottle 1 for the purpose of connecting to the latter the drainage fluid guide tube 2, has a mechanism whereby the guide tube 2 is hermetically connected to the fluid evacuator so that no gas escapes from the connection between the fluid receiving bottle 1 and the tube 2 and whereby the guide tube 2 is connected so as to be in gas and fluid communication with the interior of the wound concerned. The connector means may be arranged such that a fluid inlet port 15 is mounted at the opening of a fluid inlet seat 19 of the fluid bottle 1, and the drainage fluid guide tube 2 is inserted into the bore formed in the fluid inlet port 15. The port 15 is rigidly bonded to the opening portion of the fluid inlet seat 19 so that the connection therebetween is made air-tight. The fluid inlet port 15 has a sleeve 23 which is positioned inside the fluid receiving bottle l. The sleeve 23 is fitted into the fluid inlet seat 19, whereby the connection between the port 15 and the seat 19 is reinforced. The fluid inlet port 15 further has a projection 21 which is extended from the distal end of the sleeve 23 by 2 to 30 mm, whereby the drainage fluid which is evacuated through the guide tube 2 and the fluid inlet port 15 directly drops into the fluid receiving bottle 1 rather than flowing on the inner wall surface of the bottle 1.

The fluid inlet port 15 is provided with a stopper means. This is a means for closing off the interior of the fluid evacuator when it is set in an evacuating condition and also for preventing drainage fluid from flowing back into the wound as this would otherwise occur on the release of the negative pressure when the drainage fluid accumulated in the evacuator is discharged. In other words, the stopper means is a mechanism for selectively opening and closing the connector means so that the interior of the evacuator and that of the wound are connected such as to be in gas and fluid communication with each other only when the connector means is opened by the stopper means. For example, the stopper means is arranged such that a plate clamp 17 which is fitted on the fluid inlet port 15 is slid so that the port 15 is resiliently deformed such as to close the bore thereof.

The fluid inlet port 15 is preferably made of a resilient and soft material such as rubber or a relatively soft plastic so that the drainage fluid guide tube 2 is readily inserted thereinto but is not easily removed therefrom and so that no leak can easily occur, and further, the plate clamp 17 is readily slidable so as to work effectively. On the other hand, the plate clamp 17 is preferably made of a rigid material such as a plastic or a metal.

The arrangement shown in Fig. 2 in which the stopper means is mounted on the connector means for connecting the drainage fluid guide tube 2 to the bottle 1 is not necessarily exclusive, and the arrangement may be such that the guide tube 2 and the connector means are connected to a stopper means such as a two-way cock. Further, the stopper means may be directly connected to the guide tube 2. In other words, it is possible to employ any mechanism, provided that a tube for guiding drainage fluid from a wound can be hermetically connected to the interior of the fluid evacuator and can be readily opened and closed and easily mounted and demounted, as well as being capable of being reliably secured to the fluid inlet port 15.

The opening with a closure member for discharging body fluid is provided on the fluid receiving bottle 1. This opening is constituted by the fluid outlet port 3 of the fluid receiving bottle 1 and is adapted to be closed by a closure 7. It is only necessary for the opening to be arranged in such a manner that when the closure 7 is fitted into the fluid outlet port 3, the interior of the fluid receiving bottle 1 is hermetically sealed, while when the closure 7 is removed, the body fluid accumulated in the fluid receiving bottle 1 can be readily discharged. One end of the closure 7 is preferably fixed to the fluid receiving bottle 1 in the manner shown in Fig. 1 for the sake of convenience during use.

Further, the fluid receiving bottle 1 is provided with a connecting port 25 which is connected such as to be in gas communication with the evacuation bottle 31, detailed hereinafter, through the connecting tube 27.

The inflatable balloon 39, which is incorporated in the evacuation bottle 31, is hermetically secured to a cap 33 which is, in turn, screwed onto a thread 35 formed at the opening provided in the bottom of the evacuation bottle 31. In this case, it is preferable to employ a packing material 37 for the purpose of hermetically fitting the cap 33 to the evacuation bottle 31. If the balloon 39 is secured to the screw cap 33 in a manner such as that in the first embodiment (shown in Fig. 2), it is advantageously possible to wash the interior of the evacuation bottle 31 by removing the balloon 39 as required. It is also possible to accomplish the object of the present invention by fixing the balloon 39 directly to the evacuation chamber 82 in a manner such as that in the second embodiment shown in Fig. 3. Although in these embodiments the balloon 39 incorporated in the evacuation bottle 31 is positioned at the bottom of the bottle 31, the balloon 39 may be mounted at the top or on the side surface of the bottle 31 without leading to any functional obstruction.

The interior 38 of the balloon 39 is kept in constant communication with the atmosphere through a vent 34 alone. The vent 34 is inlet and outlet port for air which is required so as to allow the passage of air when the balloon 39 inflates and deflates. The diameter of the vent 34 is not limited to a particular value but is preferably set at 5 mm or less.

The evacuation bottle 31 is provided with an evacuating means for reducing the pressure within the bottle 31. The evacuating means comprises a hollow resilient member (a rubber bulb 41 in this embodiment), and two one-way valves 47 and 53 mounted on the resilient member. The evacuating means is rigidly secured to an air inlet port 43 of the evacuation bottle 31. The configuration of the hollow resilient member is not particularly limited, and the resilient member may be spherical or in the form of a bellows. As to the one-way valves 47 and 53, it is possible to employ any type of valve, provided that they have a valve structure which allows gas to flow in one direction only and prevents any gas from flowing back. The valves employed in this embodiment are, therefore, not necessarily exclusive.

The one-way valve 47 connects the interior 40 of the rubber bulb 41 and the interior of the evacuation bottle 31. It is only necessary for the one-way valve 47 to be so arranged that the air inside the evacuation bottle 31 is allowed to flow only into the interior 40 of the rubber bulb 41 and that no air flows back into the interior of the evacuation bottle 31. In this embodiment, a valve connector 45 is closely attached to the air inlet port 43 of the bottle 31, and one or more bleed vents 51 are provided in the side surface of the upper cylindrical portion of the connector 45, the vents 51 being hermetically closed by a rubber band 49 at all times.

The one-way valve 53 is provided at that side surface of the rubber bulb 41 which is exposed to the atmosphere. It is only necessary for the valve 53 to be so arranged that the air in the interior 40 of the rubber bulb 41 is allowed to flow out into the atmosphere and that no air flows back into the interior 40. In accordance with this embodiment, a ball 57 is received in a flange 59 having at its lower side a vent 58 with a small diameter, and a ball stopper 55 is provided in the upper portion of the flange 59. In use, when the rubber bulb 41 is compressed manually, the rubber bulb 41 is deformed to pressurize the air in the interior 40, causing the ball 57 to be pushed upwards and thereby allowing the air to be discharged into the atmosphere. At this time, the rubber band 49 of the one-way valve 47 is pressurized by the pressurized air so as to press against the bleed vents 51, whereby the valve 47 is brought to a closed position wherein there is no risk of the air within the interior 40 flowing into the interior of the evacuation bottle 31. When the operator's fingers are removed from the rubber bulb 41, the interior 40 is expanded by virtue of the resilient recovery of the rubber bulb 41, and a negative pressure is created therein. In consequence, the ball 57 is drawn by suction so as to close the small-diameter vent 58, whereby the one-way valve 53 is closed, and the air within the interior of the evacuation bottle 31 pushes up the rubber band 49 from the bleed vents 51 and flows into the interior 40 of the rubber bulb 41 through the vents 51. Accordingly, the air within the interior of the evacuation bottle 31 is discharged into the atmosphere through the one-way valves 47 and 53 by alternately compressing and releasing the rubber bulb 41, whereby the air within the interior of the evacuation bottle 31 defined by the inner wall of the bottle 31 and the outer wall of the balloon 39 is evacuated.

The resiliency, or restoring force from a compressed state to a normal state, of a material employed for the rubber bulb 41 is set such as to be larger than the resiliency, or inflating and deflating force, of a rubber material employed for the balloon 39 so that the balloon 39 is inflated within the evacuation bottle 31 by alternately compressing and releasing the rubber bulb 41. The gas required for the balloon 39 to be inflated and deflated flows in and out from the interior of the balloon 39 through the vent 34.

The evacuation bottle 31 is further provided with a connecting port 61 which is connected with the fluid receiving bottle 1 through the connecting tube 27 such as to be in gas communication therewith. If the arrangement is such that the connecting tube 27 is hermetically and detachably inserted into the connecting port 61, the connecting tube 27 can readily be exchanged for a new one when it is necessary for the fluid receiving bottle 1 alone to be replaced, and it is advantageously possible to repeatedly use the evacuation bottle 31 including the rubber bulb 41 and other members. When two independent bottles are employed as in the case of this mebodiment (shown in Fig. 2), the fluid receiving bottle 1 and the evacuation bottle 31 are connected together through the connecting tube 27 as described above. If detachable adhesive tape is employed in the form of pieces 29 and 65 which are additionally attached to the respective opposing side surfaces of the bottles 1 and 31 and are adhered to each other, the fluid evacuator can be handled safely and becomes conveniently portable. In addition, when the fluid evacuator is carried by a patient when ambulating, the two bottles 1 and 31 may be placed in carrying bag which are suspended from the body of the patient by cord or strap.

In use of the fluid evacuator according to the present invention, the closure 7 is hermetically set into the fluid outlet port 3 of the fluid receiving bottle 1, and the drainage fluid guide tube 2 leading to a wound of a human body is inserted into the fluid inlet port 15, the lower portion of the port 15 being resiliently pressed by the plate clamp 17 so that the port 15 is closed. Further, the connecting tube 27 is inserted into the connecting port 61 of the evacuation bottle 31 so that the bottles 1 and 31 are connected such as to be in gas communication with each other. Upon completion of the above preparations, the rubber bulb 41 is alternately compressed and released by the operator's fingers. In consequence, the air within the interior of the evacuation bottle 31, together with the air within the interior of the fluid receiving bottle 1 which is connected to the former by the connecting tube 27, is discharged into the atmosphere through the one-way valves 47 and 53, so that a negative pressure is created inside the bottles 1 and 31. As the vacuum level increases, the balloon 39 inflates within the evacuation bottle 31. When the balloon 39 inflates to such an extent that it substantially fills the interior of the evacuation bottle 31, the operation of alternately squeezing and releasing the rubber bulb 41 is stopped. In this state, a negative pressure in correspondence with the resiliency of the balloon 39 is kept in the fluid evacuator. Next, the plate clamp 17 is slid so as to open the fluid inlet port 15. In consequence, the drainage fluid is evacuated from the wound through the guide tube 2 by virtue of the negative pressure produced within the bottles 1 and 31 and is dropped into the fluid receiving bottle 1. The negative pressure within the bottles 1 and 31 is maintained at a substantially constant level corresponding to the resiliency of the balloon 39 even when the quantity of received body fluid increases. Unlike conventional fluid evacuators such as simple evacuated bottles, the fluid evacuator according to the present invention involves no risk of the negative pressure linearly lowering in accordance with the increase in the quantity of body fluid accumulated. Accordingly, the present invention offers great advantages in the practice of various forms of medical treatment.

In the course of medical treatment, when the balloon 39 deflates as a result of leakage of air from the wound and the necessary suction force consequently disappears, the balloon 39 is inflated again simply by closing the fluid inlet port 15 by the plate clamp 17 and alternately compressing and releasing the rubber bulb 41, using the fingers. Thus, the fluid evacuator according to the present invention has excellent operability. Since the whole of the fluid evacuator can be controlled by a closed system at all times, it is advantageously possible to provide a safe and hygienic fluid evacuator for medical use which involves no risk of bacilli entering the same. Further, since the fluid receiving bottle 1 is independent of the suction force generator, it is conveniently possible for a person engaged in medical care to directly read off from the fluid receiving bottle 1 the quantity of drainage fluid accumulated at any time during the fluid evacuation, as required.

As has been described above, the fluid evacuator for medical use according to the present invention involves fewer of the variations in suction force which may be caused with the increase in quantity of drainage fluid being evacuated from a wound of a human body and enables an appropriate negative pressure to be applied to the wound at all times, which advantageously hastens the healing of the wound. There is no risk of any positive pressure being produced which would force the accumulated drainage fluid back into the wound. Further, the fluid evacuator can be handled easily. Since the fluid receiving zone and the negative pressure maintaining zone are separated from each other and since both the evacuation and accumulation of drainage fluid are carried out with rigid containers, it is possible to accurately and readily measure the quantity of drainage fluid collected at any time during the use of the evacuator. For most of patient conditions wherein drainage fluids need to be evacuated, there is no necessity for the accumulated drainage fluid to be discharged from the fluid evacuator according to the present invention at the time of resetting the suction force during the evacuation of drainage fluid, and this enables newly evacuated drainage fluid to be added to the received fluid. Thus, the fluid evacuator according to the present invention offers faster healing for patients as well as facilitating management.

## Claims

1. A fluid evacuator for medical use comprising:
a first chamber (1, 80) for receiving and accumulating body fluid evacuated from a wound in a human body;
a second chamber for generating therein a suction force through the resiliency of an inflatable balloon member (39) provided within a rigid container (31, 82), said first and second chambers being connected at the upper portions thereof in such a manner as to be in gas communication with each other;
connector means (15) provided on said first chamber for connecting thereto a body fluid guide tube (2);
stopper means (17) provided on said first chamber for opening and closing said connector means;
an opening (3) with a closure member (7) provided on said first chamber for discharging the body fluid accumulated in said first chamber;
said inflatable balloon member being incorporated in said second chamber such that at least one end of said balloon member is open to the atmosphere so that the interior of said balloon member and the atmosphere are in gas communication with each other; and
evacuating means provided on said second chamber for reducing the pressure within said second chamber, said evacuating means including two one-way valves (47, 53) and a hollow resilient member (41), one of said valves (47) being operably connected to said second chamber so that the gas within said second chamber flows only into the interior of said resilient member, the other valve (53) being so disposed that the gas within the interior of said resilient member flows out only to the atmosphere, the resiliency or compressive restoring force of said resilient member being larger than the resiliency or deflating force of said balloon member, whereby the air within said fluid evacuator may be expelled to the atmosphere by alternately compressing and releasing said resilient member thereby creating a negative pressure within said container and inflating said balloon member within said second chamber so that it is possible to maintain a constant negative pressure at all times by virtue of the resiliency of said balloon member.

2. A fluid evacuator for medical use according to Claim 1, wherein said first and second chambers are independently constituted by individual rigid containers (1, 31) and are connected in such a manner as to be in gas communication with each other through a detachable tubular passage (27), for enabling the individual handling of each of the two containers.

## Patentansprüche

1. Flüssigkeits-Evakuiervorrichtung für medizinischen Gebrauch, umfassend:
eine erste Kammer (1, 80) zum Aufnehmen und Sammeln von aus einer Wunde in einem menschlichen Körper abgesaugter Körperflüssigkeit,
eine zweite Kammer, in der eine Saugkraft mittels der Elastizität eines in einem starren Behälter (31, 82) vorgesehenen aufblasbaren Ballonelementes (39) erzeugbar ist, wobei die ersten und zweiten Kammern an ihren oberen Abschnitten in der Weise (miteinander) verbunden sind, daß sie in Gasaustauschbeziehung miteinander stehen,
eine an der ersten Kammer vorgesehene Anschlußeinheit (15) zum Anschließen eines Körperflüssigkeit-Führungsschlauches (2),
eine an der ersten Kammer vorgesehene Absperreinheit (17) zum Öffnen und Schließen der Anschlußeinheit,
eine an der ersten Kammer vorgesehene Öffnung (3) mit einem Verschlußstück (7) zum Austragen der in der ersten Kammer angesammelten Körperflüssigkeit,
wobei das aufblasbare Ballonelement so in der zweiten Kammer eingeschlossen ist, daß mindestens ein Ende des Ballonelements zur Außenluft hin offen ist und damit das Innere des Ballonelements und die Außenluft in Gasaustauschbeziehung miteinander stehen, und
eine an der zweiten Kammer vorgesehene Evakuiereinheit zum Reduzieren des in der zweiten Kammer herrschenden Drucks, wobei die Evakuiereinheit zwei Einwegventile (47, 53) und ein hohles elastisches Element (41) aufweist, das eine der Ventile (47) betrieblich (wirkungsmäßig) so mit der zweiten Kammer verbunden ist, daß das in der zweiten Kammer vorhandene Gas nur in das Innere des elastischen Elements einströmt, das andere Ventil (53) so angeordnet ist, daß das im Inneren des elastischen Elements vorhandene Gas nur zur Außenluft hin ausströmt, die Elastizität oder Druckrückstellkraft des elastischen Elements größer ist als die Elastizität oder (Druck)-Entleerungskraft des Ballonelements, so daß die in der Flüssigkeits-Evakuiervorrichtung enthaltene Luft durch abwechselndes Zusammendrücken und Loslassen des elastischen Elements zur Außenluft austreibbar ist und dabei im Behälter ein Unterdruck erzeugt und das Ballonelement in der zweiten Kammer aufgeblasen wird, so daß mittels der Elastizität des Ballonelements ständig ein konstanter Unterdruck aufrechterhaltbar ist.

2. Flüssigkeits-Evakuiervorrichtung für medizinischen Gebrauch nach Anspruch 1, dadurch gekennzeichnet, daß die ersten und zweiten Kammern unabhängig durch getrennte starre Behälter (1, 31) gebildet und so miteinander verbunden sind, daß sie über einen abnehmbaren rohrförmigen Durchgang (27) in Gasaustauschbeziehung miteinander stehen und damit die getrennte Handhabung jedes der beiden Behälter ermöglicht ist.

## Revendications

1. Dispositif d'évacuation de fluide pour application médicale, comprenant:

une première chambre (1, 80) destinée à recevoir et accumuler une humeur évacuée d'une blessure d'un corps humain,

une seconde chambre destinée à créer une force d'aspiration grâce à l'élasticité d'un organe gonflable (39) sous forme d'un ballon, placé dans un récipient rigide (31, 82), la première et la seconde chambre étant reliées, à leurs extrémités supérieures, afin qu'elles permettent la circulation d'un gaz entre elles,

un dispositif de raccordement (15) disposé sur la première chambre et permettant le raccordement d'un tube (2) de guidage d'une humeur,

un dispositif (17) de bouchage placé sur la première chambre et destiné à ouvrir et fermer le dispositif de raccordement,

un ouverture (3) munie d'un organe de fermeture (7) et formée sur la première chambre afin que l'humeur accumulée dans la première chambre puisse être évacuée,

l'organe gonflable en forme de ballon étant incorporé à la seconde chambre de manière qu'une extrémité au moins de l'organe en forme de ballon soit reliée à l'atmosphère et que l'intérieur de l'organe en forme de ballon et l'atmosphère communiquent en permettant la circulation d'un gaz et

un élément d'évacuation disposé sur la seconde chambre et destiné à réduire la pression dans cette seconde chambre, l'élément d'évacuation comprenant deux clapets de retenue (47, 53) et un organe élastique creux (41), l'un des clapets (47) étant raccordé à la seconde chambre de manière que le gaz de la seconde chambre ne s'écoule que vers l'intérieur de l'organe élastique, l'autre clapet (53) étant disposé de manière que le gaz de l'intérieur de l'organe élastique ne s'écoule que vers l'atmosphère, la force de rétablissement après compression ou d'élasticité de l'organe élastique étant supérieure à la force d'élasticité ou de dégonflement de l'organe en forme de ballon, si bien que l'air présent dans le dispositif d'évacuation de fluide peut être évacué vers l'atmosphère par compressions et relâchements alternés de l'organe élastique avec création d'une dépression dans le récipient et gonflage de l'organe en forme de ballon dans la seconde chambre, si bien qu'une dépression constante peut être maintenue à tout moment grâce à l'élasticité de l'organe en forme de ballon.

2. Dispositif d'évacuation de fluide pour application médicale selon la revendication 1, dans lequel la première et la seconde chambre sont constituées indépendamment par des récipients rigides individuels (1, 31) et sont reliées de manière qu'elles communiquent l'une avec l'autre en permettant la circulation d'un gaz par un passage tubulaire amovible (27), permettant la manutention individuelle de chacun des deux récipients.

FIG. 1

F I G. 2

F I G. 3